# EUROPEAN PATENT APPLICATION

(11) **EP 2 769 717 A1**
(43) Date of publication of application: **27.08.2014**
(21) Application number: 12881084.3
(22) Date of filing: 12.07.2012
(51) Int. Cl.: A61K 31/167, A61K 38/06, A61K 9/08, A61K 9/107, A61K 9/16, A61K 9/20, A61K 9/48, A61P 1/16, A61P 25/04, A61P 39/02

(54) **A COMPOSITION COMPRISING REDUCED GLUTATHIONE AND ACETAMINOPHEN AND PREPARATION METHOD THEREOF**

(71) Applicant: Hainan Wei-Kang Pharmaceutical (Qianshan) Company Limited, Anhui 246300 (CN)
(72) Inventor: WANG, Liuyi, Hefei Anhui 230000 (CN); WANG, Jincan, Anqing Anhui 246300 (CN)
(74) Representative: Svingor, Adam
(86) International application number: PCT/CN2012/078550
(87) International publication number: WO 2014/008648

(57) **Abstract**

The invention relates to a pharmaceutical composition of glutathione and acetaminophen and preparation method thereof. The active ingredients of the composition include glutathione with composition ration of 0.1% ∼ 99.9% and acetaminophen with composition ratio of 99.9% ∼ 0.1%. The further purpose of the invention is to prepare glutathione and acetaminophen composition (raw materials) into various pharmaceutically acceptable dosage forms, such as tablets, sustained/controlled release preparations, capsules, pills, syrups, films, granules, oral solutions, oral suspensions, oral emulsions and oral powders. The beneficial effects of the invention is reflected in that glutathione and acetaminophen combination can effectively prevent the liver cell damage and necrosis caused by acetaminophen overdose and is strongly in favor of cancer pain relieving and chemotherapy.

## Description

### Field of the Invention

The invention relates to a pharmaceutical composition and preparation method thereof, specifically a pharmaceutical composition of glutathione and acetaminophen and preparation method thereof.

### Description of the Prior Art

Reduced glutathione (GSH) is a mercapto tripeptide of glutamic acid, cysteine and glycine. The function of glutathione is to provide antioxidant and toxinicide activities. The sulfhydryl group of cysteine is the active group of glutathione (therefore glutathione is often shortened as G-SH) and is prone to combine with some drugs (e.g., paracetanol), toxins (e.g., free radical, iodoacetic acid, mustard gas, lead, mercury, arsenic and other heavy metals). It enjoys the function of integrated detoxification. Therefore, glutathione (especially glutathione in liver cells) can participate in biotransformation, thus transforming the harmful toxins in body into harmless substances excreted from the body. Reduced glutathione also helps to maintain normal functions of the immune system.

Acetaminophen is one of acetanilide antipyretic analgesics. It inhibits cycloxygenase and selectively inhibits the synthesis of prostaglandin from the hypothalamic thermoregulatory center, resulting in peripheral vasodilatation and sweating to achieve antipyretic effect. Its antipyretic effect is similar to aspirin, specifically inhibiting prostaglandin synthesis and release and increasing pain threshold to achieve analgesic effect. It is a peripheral analgesic, with effect weaker than aspirin, and only effective on mild to moderate pain. Acetaminophen currently is considered as the preferred antipyretic analgesic because of its fewer side effects and excellent efficacy. Acetaminophen is the most widely used drug around the world; it is not only one of the top antipyretic analgesics in international pharmaceutical market, but also one of bulk drugs with largest production in China.

The action mechanism of acetaminophen has been debated for many years and at present it is generally believed that acetaminophen works through inhibiting cycloxygenase -3 (COX-3). After taken, 90% acetaminophen is metabolized by the liver, in which 60% is combined with glucuronide and 30% with sulfuric acid to form non-toxic substances and excreted by the kidneys. 4% -5% is metabolized by liver cytochrome P450 oxidase system to generate N-acetyl-phenyl quinone imine (NAPQI), and then the latter is transformed to non-toxic substance through the covalent bonding with glutathione and then excreted by the kidneys. If ingested acetaminophen dosage is far more than therapeutic dose, glucuronidation and sulfation pathway saturation will be resulted and a huge amount of acetaminophen will be metabolized by cytochrome P450 system, leading to rapid depletion of glutathione.

When the left glutathione is less than 30% of normal amount, NAPQI not covalently bonded with it will be covalently bonded with hepatocytes macromolecules, resulting in liver cell damage and necrosis.

The combination of glutathione and acetaminophen can effectively prevent the liver cell damage and necrosis caused by acetaminophen overdose and is strongly in favor of cancer pain relieving and chemotherapy.

### Objectives and Summary of the Invention

The purpose of the invitation is to provide a composition of glutathione and acetaminophen. The active ingredients of the composition are glutathione with composition ration of 0.1% ∼ 99.9% and acetaminophen with composition ratio of 99.9% ∼ 0.1%. The composition can effectively prevent the liver cell damage and necrosis caused by acetaminophen overdose and is strongly in favor of cancer pain relieving and chemotherapy.

The further purpose of the invention is to prepare glutathione and acetaminophen composition (raw materials) into various pharmaceutically acceptable dosage forms, such as tablets, sustained/controlled release preparations, capsules, pills, syrups, films, granules, oral solutions, oral suspensions, oral emulsions and oral powders.

The invention reaches the above mentioned objectives through the following technical solution:

Glutathione and acetaminophen composition, wherein:

The active ingredients of the composition include glutathione with composition ration of 0.1% ∼ 99.9% and acetaminophen with composition ratio of 99.9% ∼ 0.1%.

The glutathione and acetaminophen composition is in pharmaceutically acceptable dosage form such as tablets, sustained/controlled release preparations, capsules, pills, syrups, films, granules, oral solutions, oral suspensions, oral emulsions and oral powders.

The tablet of glutathione and acetaminophen composition is composed of the following integrates; the ratio of each integrate amount to the total amount is described as follows:

| | |
|---|---|
| Acetaminophen: | 35% ∼ 45% |
| Reduced glutathione: | 10% ∼ 15% |
| Starch: | 5% ∼ 8% |
| Sodium dodecyl sulfate: | 0.005% ∼ 0.01% |
| Stearic acid: | 5% ∼ 10% |
| Cetyl alcohol: | 5% ∼ 10% |
| Polyvidone (PVP): | 2.5% ∼ 5% |
| Microcrystalline cellulose: | 5% ∼ 10% |
| Micropowder silica gel: | 0.6% ∼ 1% |
| Lactose: | 5% ∼ 8% |
| Talcum powder: | 3% ∼ 5% |
| 10% starch paste: | 5% ∼ 15% |
| Magnesium stearate: | 0.5% ∼ 1% |

Preparation steps:
1. dissolve glutathione into ethanol solution and dissolve PVP into glutathione and ethanol solution, add micropowder silica gel and mix it evenly, add stearic acid and cetyl alcohol, heat the solution in water bath to 60°C to melt above added substances, and then add the uniform mixture of microcrystalline cellulose, lactose and talcum powder into the melted substances and mix them round for 1h;
2. Spread above sticky mixture on a plate for 20min at room temperature, granulate the mixture with 16-mesh sieve when it becomes a clump, and then stabilize and dry the granules at 30°C;
3. Prepare 10% starch paste: dissolve sodium lauryl sulfate into purified water, add starch powder and disperse it evenly, heat the mixture to make it gelatinized, and finally generate 10% starch paste;
4. Take formulated amount of acetaminophen and evenly mix it with starch, add adequate 10% starch paste, granulate it with 16-mesh sieve, dry wet granules at 40°C ∼ 60°C, then stabilize granules with 16-mesh sieve and mix the stabilized granules with the dry granules made in Step 2, add magnesium stearate, finally press the mixture into tablets.

Another technical solution is also proposed by the invention, i.e., the composition is made into oral solution with following integrates; the ratio of each integrate amount to the total amount is described as follows

| | |
|---|---|
| Acetaminophen: | 5% ∼ 10% |
| Reduced glutathione: | 1% ∼ 4% |
| Hydroxypropyl-β- cyclodextrin: | 7% ∼ 10% |
| Propanediol: | 30% ∼ 40% |
| Glycerin: | 8% ∼ 12% |
| Sucrose: | 35% ∼ 45% |
| Sodium cyclamate: | 1% ∼ 2% |
| Saccharin sodium: | 0.05% ∼ 0.1% |
| Sodium citrate: | 0.5% ∼ 0.8% |
| Citric acid: | 0.035 ∼ 0.05% |
| Myrica rubra essence: | 0.001% ∼ 0.002% |
| Rose pigment: | 0.001% ∼ 0.003% |

Preparation steps: dissolve glutathione into 100ml water, add hydroxypropyl-β-cyclodextrin to make the solution in saturation state, then set the solution aside; weigh formulated amount of sucrose, sodium cyclamate, saccharin sodium, sodium citrate and citric acid and put them into dosing jar, add water to 500mL, heat the jar to boil the solution for 15min, filter it with 10µm microporous filtering film, put the filtered solution into one storage jar to cool down below 70°C, set the jar aside; take propanediol into dosing jar, heat it to 70°C, then add acetaminophen and make it fully dissolved at 70°C, add glycerin and mix it evenly, then add above two kinds of prepared solution into the jar, finally add myrica rubra essence and rose pigment and mix all of them evenly.

Another technical solution is also proposed by the invention, i.e., the composition is made into granules with following integrates; the ratio of each integrate amount to the total amount is described as follows:

| | |
|---|---|
| Acetaminophen: | 30% ∼ 40% |
| Reduced glutathione: | 8% ∼ 12% |
| Corn starch: | 10% ∼ 15% |
| Sodium dodecyl sulfate: | 0.03% ∼ 0.07% |
| Stearic acid: | 1.5% ∼ 2.0% |
| Cetyl alcohol: | 5% ∼ 7% |
| Polyvidone (PVP): | 5% ∼ 10% |
| Crosslinking polyvingypyrrolidone (PVPP): | 1.5% ∼ 2.0% |
| Pregelatinized starch: | 10% ∼ 20% |
| Micropowder silica gel: | 0.3% ∼ 0.8% |
| Microcrystalline cellulose: | 5% ∼ 7% |
| Lactose: | 3% ∼ 6% |
| Talcum powder: | 2% ∼ 5% |
| Magnesium stearate: | 0.2% ∼ 0.6% |

Preparation steps:
1. dissolve glutathione into 10% ethanol solution and dissolve PVP into glutathione and ethanol solution, add micropowder silica gel and mix it evenly, add stearic acid and cetyl alcohol, heat the solution in water bath to 60°C to melt above added substances, and then add the uniform mixture of microcrystalline cellulose, lactose, magnesium stearate and talcum powder into the melted substances and mix them round for 1h;
2. Spread above sticky mixture on a plate for 20min at room temperature, granulate the mixture with 16-mesh sieve when it becomes a clump, and then dry and stabilize the granules at 30°C;
3. Evenly mix acetaminophen with corn starch, prepare starch paste (used as adhesive) with pregelatinized starch and polyvidone, then evenly spray the paste on the mixture of Boiling fluidized acetaminophen and corn starch, make the mixture into wet granules through fluidized-bed granulation process at 55°C with good ventilation condition, dry those wet granules at 80°C, then cool them at 50°C to make their moisture meet the requirement and stabilize the dried granules, subsequently add stearic acid and crosslinking polyvingypyrrolidone and mix them evenly, add the stabilized granules made in Step 2, finally mix all of them evenly.

The beneficial effects of the invention is reflected in that the combination of glutathione and acetaminophen can effectively prevent the liver cell damage and necrosis caused by acetaminophen overdose and is strongly in favor of cancer pain relieving and chemotherapy.

### Detailed Description of the Invention

In order to make the technical means, creative features, purposes and effects of the invention easily understood, specific cases are listed to further comprehensively illustrate the invention.

A composition of glutathione and acetaminophen, wherein active ingredients are
Reduced glutathione with composition ration of 0.1% ∼ 99.9%, and
Acetaminophen with composition ratio of 99.9% ∼ 0.1%

### Embodiment 1 (tablet):

| | |
|---|---|
| Acetaminophen: | 30g |
| Reduced glutathione: | 9g |
| Starch: | 3g |
| Sodium dodecyl sulfate: | 0.06g |
| Stearic acid: | 6.0g |
| Cetyl alcohol: | 6.0g |
| Polyvidone (PVP): | 3.1g |
| Microcrystalline cellulose: | 5.88g |
| Micropowder silica gel: | 0.54g |
| Lactose: | 4.98g |
| Talcum powder: | 2.49g |
| 10% starch paste: | adequate |
| Magnesium stearate: | 0.47g |
| | Totally 100 tablets are prepared. |

Preparation steps:
1. dissolve glutathione into 4.32ml 10% ethanol solution and dissolve PVP into glutathione and ethanol solution, add micropowder silica gel and mix it evenly, add stearic acid and cetyl alcohol, heat the solution in water bath to 60°C to melt above added substances, and then add the uniform mixture of microcrystalline cellulose, lactose and talcum powder into the melted substances and mix them round for 1h;
2. Spread above sticky mixture on a plate for 20min at room temperature, granulate the mixture with 16-mesh sieve when it becomes a clump, and then stabilize and dry the granules at 30°C;
3. Prepare 10% starch paste: dissolve 0.06g sodium lauryl sulfate into about 20ml purified water, add about 2g starch powder and disperse it evenly, heat the mixture to make it gelatinized, finally generate 10% starch paste;
4. Take formulated amount of acetaminophen and evenly mix it with starch, add adequate 10% starch paste, granulate it with 16-mesh sieve, dry wet granules at 40°C ~ 60°C, then stabilize granules with 16-mesh sieve and mix the stabilized granules with the dry granules made in Step 2, add magnesium stearate, finally press the mixture into tablets.

### Embodiment 2 (oral solution):

| | |
|---|---|
| Acetaminophen: | 24g |
| Reduced glutathione: | 7.2g |
| Hydroxypropyl-β- cyclodextrin: | 27.7g |
| Propanediol: | 250mL |
| Glycerin: | 50mL |
| Sucrose: | 300g |
| Sodium cyclamate: | 8g |
| Saccharin sodium: | 0.5 g |
| Sodium citrate: | 2.9 g |
| Citric acid: | 0.4 g |
| Myrica rubra essence: | 1 ml |
| Rose pigment: | 1g |
| | Totally 100 pieces are prepared. |

Dissolve 7.2g glutathione into 100ml water, add 27.7g hydroxypropyl-β-cyclodextrin to make the solution in saturation state, then set the solution aside; weigh 300g sucrose, 8g sodium cyclamate, 0.5g saccharin sodium, 2.9g sodium citrate and 0.4g citric acid and put them into dosing jar, add water to 500mL, heat the jar to boil the solution for 15min, filter it with 10µm microporous filtering film, put the filtered solution into one storage jar to cool down below 70°C, set the jar aside; take 250ml propanediol into dosing jar, heat it to 70°C, then add 24g acetaminophen and make it fully dissolved at 70°C, add 50ml glycerin and mix it evenly, then add above two kinds of prepared solution into the jar and make them mixed evenly, finally add 1ml myrica rubra essence and 1g rose pigment and mix all of them evenly.

### Embodiment 3 (granule):

| | |
|---|---|
| Acetaminophen: | 30g |
| Reduced glutathione: | 9g |
| Corn starch: | 12.05g |
| Sodium dodecyl sulfate: | 0.06g |
| Stearic acid: | 1.72g |
| Cetyl alcohol: | 6.0g |
| Polyvidone (PVP): | 7.2g |
| Crosslinking polyvingypyrrolidone (PVPP): | 1.72g |
| Pregelatinized starch: | 13.05g |
| Micropowder silica gel: | 0.54g |
| Microcrystalline cellulose: | 5.88g |
| Lactose: | 4.98g |
| Talcum powder: | 2.49g |
| Magnesium stearate: | 0.47g |
| | Totally 100 bags are prepared. |

Preparation steps:
1. dissolve glutathione into 4.32ml 10% ethanol solution and dissolve PVP into glutathione and ethanol solution, add micropowder silica gel and mix it evenly, add stearic acid and cetyl alcohol, heat the solution in water bath to 60°C to melt above added substances, and then add the uniform mixture of microcrystalline cellulose, lactose, magnesium stearate and talcum powder into the melted substances and mix them round for 1h;
2. Spread above sticky mixture on a plate for 20min at room temperature, granulate the mixture with 16-mesh sieve when it becomes a clump, and then dry and stabilize the granules at 30°C;
3. Evenly mix acetaminophen with corn starch, prepare starch paste (used as adhesive) with pregelatinized starch and polyvidone, then evenly spray the paste on the mixture of Boiling fluidized acetaminophen and corn starch, make the mixture into wet granules through fluidized-bed granulation process at 55°C with good ventilation condition, dry those wet granules at 80°C, then cool them at 50°C to make their moisture meet the requirement and stabilize the dried granules, subsequently add stearic acid and crosslinking polyvingypyrrolidone and mix them evenly, add the stabilized granules made in Step 2, finally mix all of them evenly.

### Experimental Data:

1. Materials: healthy male Kunming mice (weight: 20-22g)

### 2. Methods and Results

Divide 50 mice into 5 groups: Control Group, Model Group, Embodiment Group 1, Embodiment Group 2 and Embodiment Group 3; 10 mice for each group. The mice are fed freely two days and fasted 16h before the experiment. Experimental treatment: the mice respectively of Control Group, Model Group, Embodiment Group 1, Embodiment Group 2 and Embodiment Group 3 are separately given normal saline, acetaminophen solution (0.5g/kg), tablets (0.5g/kg) made in Embodiment 1, oral solution (0.5g/kg) made in Embodiment 2, and granules made in Embodiment 3 by gavage. The mice are treated continuously 4d at intervals of 24h. The mice of control group are given 1% sodium carboxymethylcellulose (0.15g/kg) by gavage 1h after last treatment and sacrificed by cervical dislocation after 24h.

Preparation of blood serum: take blood from mouse carotid artery, stand the blood, then make it centrifuged at 3,000r/min for 15min to separate out the serum, and put the serum in refrigerator (-20°C) for storage.

Measuring indicator and method: take the prepared serum on Sigma95308 automatic biochemistry analyzer to determine the activity of aspartate transaminase (AST/GOT) and alanine aminotransferase (ALT/GPT).

### Impact of embodiments on liver damage

| Group Name | AST/IU·L⁻¹ | ALT/IU·L⁻¹ |
|---|---|---|
| Control Group | 61.3±12.2 | 40.7±7.8 |
| Model Group | 245.6±18.3^{##} | 87.8±9.5^{##} |
| Embodiment Group 1 | 65.2±12.9^{**} | 43.1±9.1^{*} |
| Embodiment Group 2 | 68.0±11.7^{**} | 45.8±9.2^{**} |
| Embodiment Group 3 | 66.0±8.6^{**} | 45.2±9.4^{*} |

| | | |
|---|---|---|
| Compared with control group, ^{#}P<0.05 and ^{##}P<0.01; compared with model group, ^{*}P<0.05 and ^{**}P<0.01 | | |

These results show that high dosage of acetaminophen and glutathione composition almost is harmless to the liver.

The technicians of the art should understand that the invention is not limited to the embodiments above. The purpose of embodiments and details description is only to illustrate the principles of the invention. According to the objectives and scope of the invention, there will be various changes and improvements, for example, the composition will be made in other pharmaceutically acceptable dosage forms including sustained/controlled release preparations, capsules, pills, syrups, films, oral suspensions, oral emulsions and oral powders. The changes and improvements will be included in the claimed scope of the invention. The claimed scope of the invention is determined by the appended claims and their equivalents.

## Claims

1. A glutathione and acetaminophen composition, comprising active ingredients:
Glutathione with composition ratio of 0.1% ~ 99.9%, and
Acetaminophen with composition ratio of 99.9% ∼ 0.1%
The glutathione and acetaminophen composition is in pharmaceutically acceptable dosage form such as tablets, sustained/controlled release preparations, capsules, pills, syrups, films, granules, oral solutions, oral suspensions, oral emulsions and oral powders.

2. The composition according to Claim 1, wherein the tablet of glutathione and acetaminophen composition is composed of the following integrates; the ratio of each integrate amount to the total amount is described as follows:
| | |
|---|---|
| Acetaminophen: | 35% ∼ 45% |
| Reduced glutathione: | 10% ∼ 15% |
| Starch: | 5% ∼ 8% |
| Sodium dodecyl sulfate: | 0.005% ∼ 0.01% |
| Stearic acid: | 5% ∼ 10% |
| Cetyl alcohol: | 5% ∼ 10% |
| Polyvidone (PVP): | 2.5% ∼ 5% |
| Microcrystalline cellulose: | 5% ∼ 10% |
| Micropowder silica gel: | 0.6% ∼ 1% |
| Lactose: | 5% ∼ 8% |
| Talcum powder: | 2.5% ∼ 5% |
| 10% starch paste: | 5% ∼ 15% |
| Magnesium stearate: | 0.5% ∼ 1% |

3. The composition according to Claim 2, wherein the tablets of glutathione and acetaminophen composition are prepared through the following steps:
a. Dissolve glutathione into ethanol solution and dissolve PVP into glutathione and ethanol solution, add and evenly mix micropowder silica gel, add stearic acid and cetyl alcohol, heat the solution in water bath to 60°C to melt above added substances, and then add the uniform mixture of microcrystalline cellulose, lactose and talcum powder into the melted substances and mix them round for 1h;
b. Spread above sticky mixture on a plate for 20min at room temperature, granulate the mixture with 16-mesh sieve when it becomes a clump, and then stabilize and dry the granules at 30°C;
c. Prepare 10% starch paste: dissolve sodium lauryl sulfate into purified water, add starch powder and disperse it evenly, heat the mixture to make it gelatinized, and finally generate 10% starch paste;
d. Take formulated amount of acetaminophen and evenly mix it with starch, add adequate 10% starch paste, granulate it with 16-mesh sieve, dry wet granules at 40°C ~ 60°C, then stabilize granules with 16-mesh sieve and mix the stabilized granules with the dry granules made in Step 2, add magnesium stearate, and finally press the mixture into tablets.

4. The composition according to Claim 1, wherein the oral solution of glutathione and acetaminophen composition is composed of the following integrates; the ratio of each integrate amount to the total amount is described as follows:
| | |
|---|---|
| Acetaminophen: | 5% ∼ 10% |
| Reduced glutathione: | 1% ∼ 4% |
| Hydroxypropyl-β- cyclodextrin: | 7% ∼ 10% |
| Propanediol: | 30% ∼ 40% |
| Glycerin: | 8% ∼ 12% |
| Sucrose: | 35% ∼ 45% |
| Sodium cyclamate: | 1% ∼ 2% |
| Saccharin sodium: | 0.05% ∼ 0.1 % |
| Sodium citrate: | 0.5% ∼ 0.8% |
| Citric acid: | 0.035 ∼ 0.05% |
| Myrica rubra essence: | 0.001% ∼ 0.002% |
| Rose pigment: | 0.001% ∼ 0.003% |

5. The composition according to Claim 4, wherein the oral solution of glutathione and acetaminophen composition is prepared through following steps: dissolve glutathione into 100ml water, add hydroxypropyl-β-cyclodextrin to make the solution in saturation state, then set the solution aside; weigh formulated amount of sucrose, sodium cyclamate, saccharin sodium, sodium citrate and citric acid and put them into dosing jar, add water to 500mL, heat the jar to boil the solution for 15min, filter it with 10µm microporous filtering film, put the filtered solution into one storage jar to cool down below 70°C, set the jar aside; take propanediol into dosing jar, heat it to 70°C, then add acetaminophen and make it fully dissolved at 70°C, add glycerin and mix it evenly, then add above two kinds of prepared solution into the jar, finally add myrica rubra essence and rose pigment and mix all of them evenly.

6. The composition according to Claim 1, wherein the granules of glutathione and acetaminophen composition are composed of the following integrates; the ratio of each integrate amount to the total amount is described as follows:
| | |
|---|---|
| Acetaminophen: | 30% ∼ 40% |
| Reduced glutathione: | 8% ∼ 12% |
| Corn starch: | 10% ∼ 15% |
| Sodium dodecyl sulfate: | 0.03% ∼ 0.07% |
| Stearic acid: | 1.5% ∼ 2.0% |
| Cetyl alcohol: | 5% ∼ 7% |
| Polyvidone (PVP): | 5% ∼ 10% |
| Crosslinking polyvingypyrrolidone (PVPP): | 1.5% ∼ 2.0% |
| Pregelatinized starch: | 10% ∼ 20% |
| Micropowder silica gel: | 0.3% ∼ 0.8% |
| Microcrystalline cellulose: | 5% ∼ 7% |
| Lactose: | 3% ∼ 6% |
| Talcum powder: | 2% ∼ 5% |
| Magnesium stearate: | 0.2% ∼ 0.6% |

7. The composition according to Claim 6, wherein the granules of glutathione and acetaminophen composition are prepared through the following steps:
a. Dissolve glutathione into 10% ethanol solution and dissolve PVP into glutathione and ethanol solution, add micropowder silica gel and mix it evenly, add stearic acid and cetyl alcohol, heat the solution in water bath to 60°C to melt above added substances, and then add the uniform mixture of microcrystalline cellulose, lactose and talcum powder into the melted substances and mix them round for 1h;
b. Spread above sticky mixture on a plate for 20min at room temperature, granulate the mixture with 16-mesh sieve when it becomes a clump, and then stabilize and dry the granules at 30°C;
c. Evenly mix acetaminophen with corn starch, prepare starch paste (used as adhesive) with pregelatinized starch and polyvidone, then evenly spray the paste on the mixture of Boiling fluidized acetaminophen and corn starch, make the mixture into wet granules through fluidized-bed granulation process at 55°C with good ventilation condition, dry those wet granules at 80°C, then cool them at 50°C to make their moisture meet the requirement and stabilize the dried granules, subsequently add stearic acid and crosslinking polyvingypyrrolidone and mix them evenly, add the stabilized granules made in Step 2, finally mix all of them evenly.
